# EUROPEAN PATENT APPLICATION

(11) **EP 2 228 002 A1**
(43) Date of publication of application: **15.09.2010**
(21) Application number: 10250434.7
(22) Date of filing: 09.03.2010
(51) Int. Cl.: A61B 1/00, A61B 1/018

(54) **Side viewing endoscope system**

(30) Priority: 09.03.2009 JP 2009054458; 09.03.2009 JP 2009054459; 03.04.2009 JP 2009090779; 15.07.2009 JP 2009166360
(71) Applicant: FUJIFILM Corporation, Tokyo (JP)
(72) Inventor: Ohki, Tomohiro, Saitama-shi, Saitama (JP); Iyama, Shozo, Saitama-shi, Saitama (JP)
(74) Representative: Stevens, Jason Paul

(57) **Abstract**

A side viewing endoscope system includes a mother endoscope (1) of a side viewing type, having an instrument raising chamber (14) including an instrument raiser member (15) for raising up or sinking down of a treatment instrument, and a biopsy channel (13) provided in an axial direction of the insertion section (3) on the endoscope and in communication with the instrument raising chamber. A daughter endoscope (100) of the front viewing type, with a slender insertion section (102), can be inserted into the biopsy channel of the mother endoscope to a window (52, 53) formed in the rigid distal end portion (3a) of the mother endoscope. Means can be provided to ensure that the instrument raiser member (15) does not interfere with the slender insertion section (102), and to ensure that the slender insertion section (102) does not project from the rigid distal end portion (3a).

## Description

The present invention relates to a side viewing endoscope system which can ensure a front visual field when an insertion section of a front viewing endoscope is inserted into an insertion path in a side viewing endoscope, which has an observation visual field in a direction generally perpendicular to the axis of the insertion section.

Typically, an endoscope has an insertion section connected to a manipulation section. The insertion section is formed from a flexible portion which is connected to the manipulation section, a movable flexing portion constituting a distal end portion of the flexible portion, and a rigid distal end portion at a distal end of the movable flexing portion. At the distal end is disposed an endoscopic observation means including an illumination portion and an observation portion, which is provided with a biopsy channel through which treatment instruments such as forceps are inserted. The treatment instrument is led out from the biopsy channel toward the direction of the observation field by the endoscopic observation means. One type of endoscope has an observation field directed in the axial direction of the insertion section, and an endoscope of this type is referred to as a front viewing endoscope; another type of endoscope has an observation field directed substantially orthogonal to the axis of the insertion section, and this is referred to as a side viewing endoscope.

In the case of the side viewing endoscope, a flat portion is formed on a side face of the rigid distal end portion of the insertion section, and an illumination window and an observation window are disposed in this flat portion. An instrument raising chamber for leading out the treatment instrument is also formed in this flat portion. An end of a light guide faces the illumination window in the side face of the rigid distal end portion, and a solid-state image pickup means is attached to the observation window. The light guide has flexibility in a bending direction, and a signal cable leading from the solid-state image pickup means also has flexibility in the bending direction. Therefore, they are bent at approximately 90 degrees in the rigid distal end portion and extend from the insertion section to the manipulation section.

The biopsy channel is a flexible path from the manipulation section to the axial direction of the insertion section. In the rigid distal end portion, a space communicating with an instrument raising chamber opened in the side face is formed, and a instrument raiser member is attached to this space. This space is therefore a treatment instrument raising space, and the biopsy channel which extends from the manipulation section opens into this treatment instrument raiser chamber. The instrument raiser member guides the treatment instrument which is inserted into the biopsy channel toward the instrument raising chamber, and is capable of carrying out a raising operation in order to aim the distal end of the treatment instrument at a treatment portion. The operation of the instrument raiser member can be performed by remote control from the manipulation section, and thus, raiser operating means including an operation lever or the like is provided on the manipulation section.

The side viewing endoscope provided with a raising mechanism for the treatment instrument is substantially configured as described above, and a construction of this type of side viewing endoscope is disclosed in Japanese Unexamined Patent Application Publication No. 2007-136044, for example. Here, the side viewing endoscope is suitable for use as a duodenoscope. The insertion section is inserted to the duodenum, the endoscopic observation means is arranged opposing the papilla, and examination and appropriate treatments are conducted. Treatment instruments used with the duodenoscope include a biliary cannulation tube, a metal stent and the like, and the treatment instrument is inserted into the duct tract through the papilla. In this way, by disposing a instrument raiser member capable of standing up and down at the rigid distal end portion, the treatment instrument can be directed to a targeted portion smoothly and reliably.

Various treatment instruments can be inserted into a side viewing endoscope, and one configured so that a slender endoscope capable of insertion into the biliary tract is inserted therein is disclosed in Japanese Unexamined Patent Application Publication No. 11-42207. That is, a contrast medium tube is inserted into the biliary tract and a contrast medium is injected therein, and then, a slender endoscope consisting of a fibre scope is led out of the instrument raising chamber through the biopsy channel and inserted into the biliary tract so that an examination is made in the biliary tract.

When the insertion section of the endoscope is inserted to the duodenum, the portion goes through the stomach from the oesophagus, but this insertion path has a considerable length. Moreover, the insertion path is bent in a complicated way and has an uneven structure having a constrictive portion and an expanded portion in the middle of the path. Thus, an insertion operation of the insertion section should be conducted carefully while its insertion direction is checked. In the side viewing endoscope, since the observation visual field is oriented to the direction orthogonal to the axis of the insertion section, the front in the insertion direction cannot be contained in the visual field by the endoscopic observation means. Since the endoscope has an observation visual field on the side, the front view of the distal end portion of the insertion section can be grasped to some degree, but the front view cannot be directly checked. Therefore, there is some difficulty in an operation to insert the insertion section, the insertion operation requires skills, and it is likely that the insertion operation takes time. In short, the side viewing endoscope has problems to be solved from the point of view of insertion operability.

The present invention was made in view of the above problems, and it is an object of at least the preferred embodiments thereof to provide a side viewing endoscope system with a simple construction that can ensure a front visual field in the insertion direction during an insertion operation.

It is also an object of at least the preferred embodiments of the present invention to provide a side viewing endoscope system in which, during insertion of an endoscope, the front visual field is ensured through the treatment instrument insertion channel, and after the insertion section is arranged at a predetermined position, the treatment instrument insertion channel is can be used for its original function.

According to the present invention, in order to solve the above-stated objectives, there is provided a side viewing endoscope system comprising: a mother endoscope of a side viewing type in which an observation means having an illumination portion and an observation portion is disposed on a side surface of a rigid distal end portion of an insertion section, a instrument raising chamber including an instrument raiser member for raising up or sinking down of a treatment instrument is formed in said rigid distal end portion, a biopsy channel provided in an axial direction of said insertion section being in communication with said instrument raising chamber; and a daughter endoscope having a slender insertion section which can be inserted into said biopsy channel of said mother endoscope and having a viewing field in a forward direction of a distal end of said slender insertion section; wherein a window is formed at said rigid distal end portion of said mother endoscope having a viewing field toward the forward direction of said biopsy channel; and said distal end portion of said daughter endoscope may be introduced through said biopsy channel into a position facing said window but not protruding from the outer surface of said rigid distal end of said mother endoscope.

As described in the above-mentioned Japanese Unexamined Patent Application Publication No. 11-42207, irrespective of whether the endoscope is an optical endoscope or an electronic endoscope, it comes into wide use that an endoscope has an insertion section which can be inserted into a biopsy channel of a side viewing endoscope. In the present invention, the side viewing type endoscope is constituted as a mother endoscope, and a daughter endoscope of a front viewing type having a slender insertion section is adapted to be inserted into the biopsy channel of the mother endoscope. By means of this construction, the mother endoscope can have an observation field for the advanced route of a body cavity by means of the slender insertion section of the daughter endoscope which is inserted into the biopsy channel of the mother endoscope.

The instrument raising chamber is constituted by a recessed portion which is open to a side face portion of the rigid distal end portion and has a treatment instrument base attached inside. Also, at a front position of the biopsy channel in the instrument raising chamber, a front end wall is located. Even if the daughter endoscope is introduced into the instrument raising chamber through the biopsy channel as an insertion path of the daughter endoscope, the front visual field cannot be ensured in that state. Thus, a window is formed in this front end wall. At least a portion of the distal end side of the rigid distal end portion can be configured by a transparent member, but the rigid distal end portion is usually configured by a non-transparent member, and in this case, the window is formed by forming a through hole in the front end wall of the rigid distal end portion.

In the instrument raising chamber, the instrument raiser member is arranged between the front end of the biopsy channel and the distal end portion of the rigid distal end portion in which the window is formed. Since the instrument raiser member is intended to change the direction of the treatment instrument inserted into the biopsy channel to the instrument raising chamber, the slender insertion section of the daughter endoscope cannot be advanced further to the window in a general side viewing endoscope. Thus, in order that the slender insertion section can pass through the position of the instrument raiser member, a daughter endoscope passing mechanism is disposed on the instrument raiser member.

The instrument raiser member is adapted to change a direction of a treatment instrument, and the treatment instrument is not inserted before the insertion section of the mother endoscope reaches the area to be examined or observed, such as the duodenum. The treatment instrument is only inserted when the examination or observation is to be performed, and at this point in time, the front visual field is no longer needed. Thus, the daughter endoscope passing mechanism provided on the instrument raiser member can be constituted such that the instrument raiser member is retreated from the treatment insertion path and the front of the biopsy channel is opened. That is, the instrument raiser member has an operation range from a sunken down position (a minimum angle) to a raised up position (a maximum angle), and the instrument raiser member can be forcedly displaced to a position beyond the minimum angle.

The instrument raiser member is normally connected to a rotatable shaft, and the rotatable shaft is supported by a wall portion of the rigid distal end portion. The instrument raiser member can be moved to perform an operation by rotating the rotatable shaft. Even in the most sunken down state, that is, at the minimum angular position of the instrument raiser member, a guide surface thereof can be led out from the instrument raising chamber. On the other hand, in the most raised up state, that is, at the maximum angular position, the instrument raising chamber is partially covered by the instrument raiser member. Specifically, in a case where a direction orthogonal to the axis of the insertion section is defined as 90 degrees, generally a treatment instrument can be bent from an angle of less than 90 degrees (inclined toward the forward direction) to an angle of more than 90 degrees (inclined toward the rearward direction).

An operation of the instrument raiser member is performed on the manipulation section side. Therefore, on the manipulation section, a raiser member operation means such as an operation lever or an operation knob is provided, or a slide lever or the like can be constituted as the raising member operation means. If an operation lever or operation knob is to be used, it can be made as an independent construction for operation of the raiser member, and since an operating member which forms the bending operation means for the insertion section is attached to the manipulation section, the lever or knob may be disposed coaxially with the rotational shaft of the operating member. By configuring the bending operating means and the raising member operation means such that they are disposed coaxially, the operating mechanism can be simplified. A transmission member such as an operating wire is connected to the raising member operation means, and the transmission means extends to the distal end of the insertion section. The operating wire can be directly connected to the instrument raiser member, but it may also be so configured that the lever is connected to the rotatable shaft and the transmission member is connected to the lever, by which the instrument raiser member can be raised up and can be operated smoothly with a slight load.

The raising member operation means is capable of switching between operation modes. That is, the instrument raiser member can be rotated from the minimum angular position to the maximum angular position in an operation range of the raiser member, which is a raising operation mode. Further, the instrument raiser member is moved to the retreated position by being set at an angular state smaller than the minimum angular position, and this is referred to as an "ex-boundary operation mode". The operation means is capable of switching between the raising operation mode and the ex-boundary operation mode. In a usual state, an operation range limiting portion is provided for limiting the range to a raising operation mode operation range, and the operation mode can be changed over by removing the limitation to the above-mentioned operation range limiting portion.

The daughter endoscope passing mechanism disposed on the instrument raiser member may be configured by a daughter endoscope passage formed on a guide surface of the instrument raiser member. That is, a through hole or a guide groove through which the slender insertion section of the daughter endoscope can be inserted may be disposed in the instrument raiser member. The through hole or guide groove is formed at an extended position of the biopsy channel. The daughter endoscope passage can be kept usually open or otherwise may have a shutter to be opened and closed by operation. If the daughter endoscope passage is usually kept open, the slender insertion section is passed through the daughter endoscope passage when the slender insertion section of the daughter endoscope is introduced therein, while a treatment instrument can be guided toward the instrument raising chamber along the guide surface of the instrument raiser member when the treatment instruction is introduced therein. For that purpose, the daughter endoscope passage should have a diameter smaller than an outer diameter of a usual treatment instrument to be inserted in a side viewing endoscope and also should have a diameter so that the slender insertion section can be passed through the through hole or guide groove.

A through hole is formed in the front end wall of the rigid distal end portion of the insertion section of the mother endoscope as a window. By the above-mentioned construction, the slender insertion section of the daughter endoscope should not be allowed to pass through the through hole, or at least not be allowed to protrude from the rigid distal end portion. Thus, a transparent plate can be positioned in the through hole to act as a stopper. As a result, if the slender insertion section of the daughter endoscope is inserted through the biopsy channel, the distal end of the slender insertion section comes into contact with the transparent plate and is stopped at the position of the transparent plate so that the slender insertion section does not protrude from the rigid distal end portion.

By making a diameter of the through hole smaller than the outer diameter of the slender insertion section with the smallest diameter of a normal daughter endoscope, the through hole can function as a restriction portion such that the slender insertion section does not pass through the through hole nor protrude from the rigid distal end portion. For example, the through hole can be made to have one or more steps, and its most reduced diameter acts as the restriction portion. If the slender insertion section is smaller than the diameter of the through hole, medical tape may be wound around an outer circumference of the distal end portion of the slender insertion section so as to increase the outer diameter thereof so that the slender insertion section is prevented from passing through the through hole. The through hole may have a uniform diameter but can be also constituted as a tapered hole whose diameter is continuously reducing toward the front (distal) side. In this case, the tapered hole has a introductory function allowing insertion of the daughter endoscope, and also has a centring function of the slender insertion section with respect to the through hole.

The daughter endoscope may be an optical endoscope, or otherwise the daughter endoscope may be constituted by an electronic endoscope when the mother endoscope is an electronic endoscope. During insertion of the mother endoscope into the body cavity, observation of the visual field of the mother endoscope, which is directed substantially orthogonal to the axis of the insertion section, is not needed, and the front visual field by the daughter endoscope is used for the purposes of insertion of the mother endoscope. A front-view observation image can be attained by the observation portion of the daughter endoscope in the mother endoscope by displaying it on a monitor screen. When the daughter endoscope is separated from the mother endoscope, the biopsy channel is left open for inserting a treatment instrument, also the monitor screen is shifted to display a side-view observation image from the mother endoscope automatically or by a manual operation.

As mentioned above, by inserting the slender front-viewing endoscope into the biopsy channel, a forward field of vision can be attained with a simple construction through a window disposed at the rigid distal end portion of the mother endoscope. Moreover, when the biopsy channel is to be used for its original purpose, that is, when a treatment instrument is to be inserted, there is no need to ensure the forward visual field, therefore, the daughter endoscope can be removed beforehand from the mother endoscope.

Embodiments of the present invention will be described referring to the attached drawings. However, the present invention is not to be interpreted as being limited to the embodiments illustrated below. In the drawings:
Fig. 1 is an explanatory diagram illustrating a mother endoscope and a daughter endoscope;
Fig. 2 is a perspective view of a distal end portion of an insertion section of the mother endoscope according to a first embodiment of the present invention;
Fig. 3 is a longitudinal sectional view of Fig. 2;
Fig. 4 is a sectional view taken along the line X-X in Fig. 3;
Fig. 5 is a diagram illustrating a construction of an operating mechanism of a instrument raiser member;
Fig. 6 is a sectional view of a raiser operating means illustrated with a bending operation means;
Fig. 7 is a sectional view of a distal end portion of an insertion section of a daughter endoscope;
Fig. 8 is an explanatory diagram illustrating a construction of the mother endoscope and the daughter endoscope as electronic endoscopes in which images from both endoscopes may be switched between in display;
Fig. 9 is a sectional view similar to Fig. 3 illustrating a state in which the daughter endoscope is incorporated in the mother endoscope;
Fig. 10 is a sectional view of a state in which the daughter endoscope is incorporated in the mother endoscope, according to a second embodiment of the present invention;
Fig. 11 is a front view of a instrument raiser member in Fig. 10 in a state in which the daughter endoscope and a treatment instrument are inserted;
Fig. 12 is a diagram illustrating a variation of the second embodiment of the present invention, in which a light source for the daughter endoscope is disposed on the mother endoscope;
Fig. 13 is a sectional view similar to Fig. 10 illustrating a variation of the daughter endoscope of the second embodiment of the present invention;
Fig. 14 is a sectional view of a part illustrating a variation of a regulation portion to prevent an insertion section of the daughter endoscope from protruding from the rigid distal end portion;
Fig. 15 is a front view of Fig. 14; and
Fig. 16 is a sectional view of a part illustrating another variation of the regulation portion to prevent the insertion section of the daughter endoscope from protruding from the rigid distal end portion.

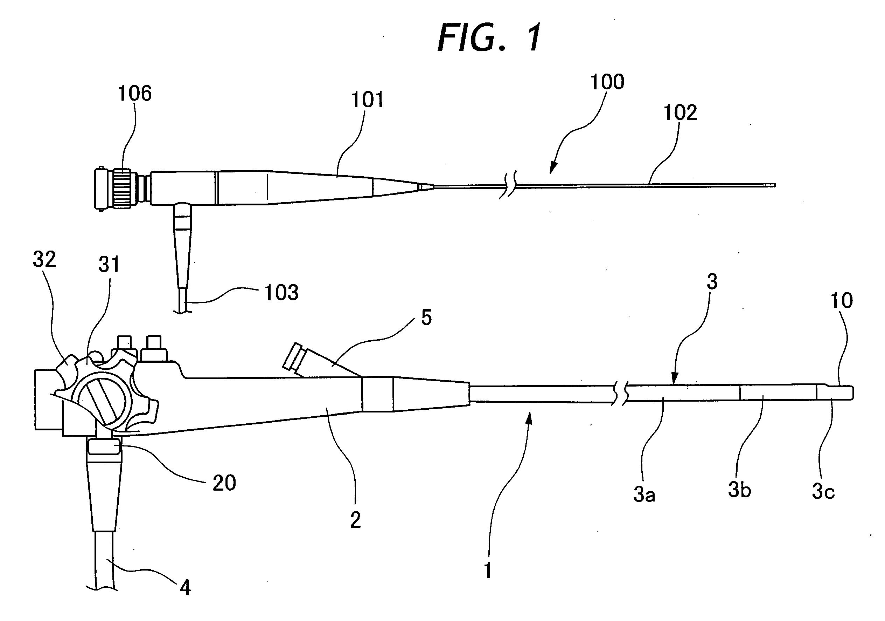

Embodiments of the present invention will be described below with reference to the attached drawings. First, Fig. 1 shows a side viewing endoscope and a thinner front viewing endoscope which can be inserted through a biopsy channel of the side viewing endoscope. Here, in the following explanation, the side viewing endoscope is referred to as a mother endoscope 1, while the front viewing endoscope to be used with the mother endoscope 1 is referred to as a daughter endoscope 100.

In the mother endoscope 1, an insertion section 3 is connected to a manipulation section 2, and a universal cord 4 is also connected to the manipulation section 2. The insertion section 3 includes a flexible portion 3a which forms most of the insertion section extending from the manipulation section 2 and which can bend in an arbitrary direction along an insertion passage of the endoscope. A distal end portion of the flexible portion 3a is a movable flexing portion 3b, and a rigid distal end portion 3c is provided at a distal end of the movable flexing portion 3b.

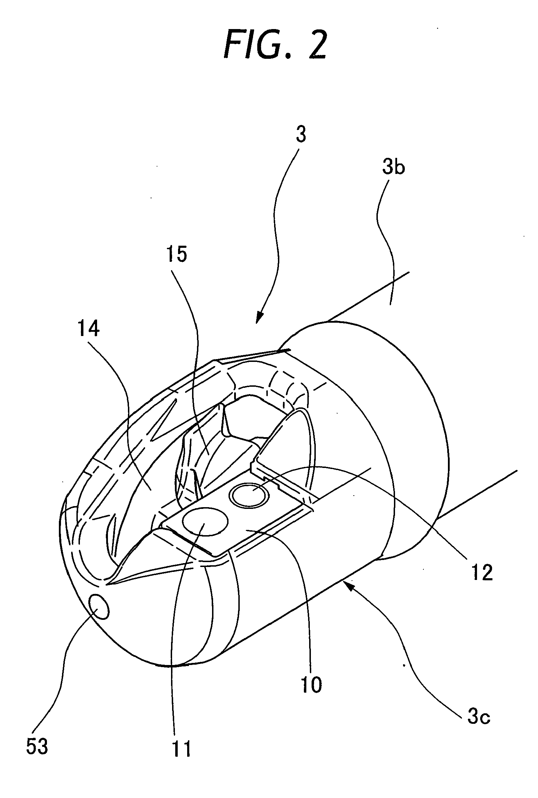

Fig. 2 shows the distal end portion of the insertion section 3 of the mother endoscope 1. The rigid distal end portion 3c which is connected to the movable flexing portion 3b has a flat face portion 10 formed in the side surface, and an illumination portion 11 and an observation portion 12 are provided as observation means in this flat face portion 10. An illumination lens is attached to the illumination portion 11, and an end face of a light guide is placed opposed to the illumination lens. An objective optical system is disposed in the observation portion 12, and a solid-state image pickup means is provided at an image forming position of the objective optical system. Since specific constructions of the illumination portion 11 and the observation portion 12 are already known, illustration and detailed description thereof will be omitted.

Here, a side viewing endoscope as the mother endoscope 1 is used mainly as a duodenoscope; this type of endoscope is inserted through a mouth to a duodenum through an oesophagus and a stomach. In a cavity wall portion of the duodenum, a papilla communicating with a biliary tract is opened. Therefore, a treatment instrument such as a cannulation tube, a stent and the like can be inserted through the papilla into the biliary tract or through the biliary tract into a common bile duct or the like so that predetermined examinations, treatments and the like may be performed. For that purpose, an insertion path for the above-mentioned treatment instrument is disposed in the side viewing endoscope.

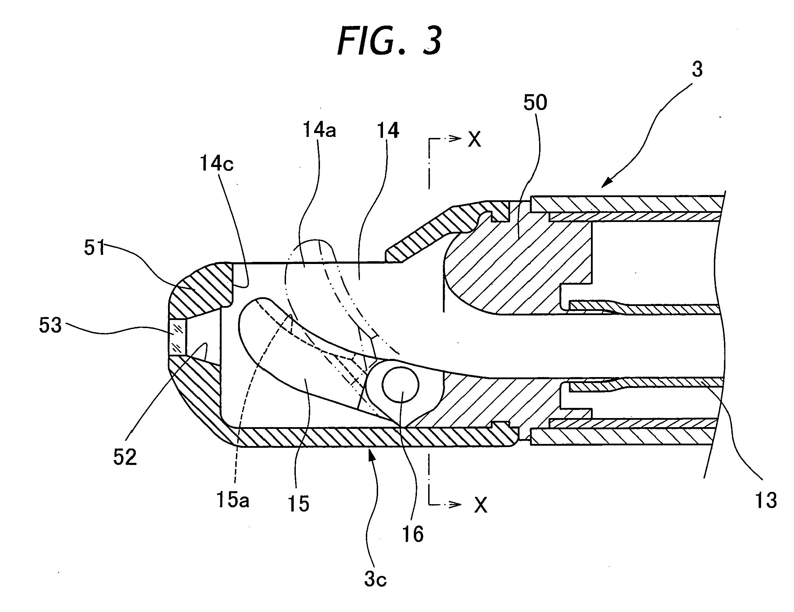

As an inlet portion in the insertion path for the treatment instrument, as shown in Fig. 1, a treatment instrument introduction portion 5 is disposed on the manipulation section 2. A construction of the rigid distal end portion 3c in the insertion section 3 is shown in Fig. 3. In this figure, reference numeral 13 denotes a biopsy channel constituted by a flexible tube, and this biopsy channel 13 extends from the manipulation section 2 through the insertion section 3. The biopsy channel 13 extends in the axial direction in the insertion section 3 and is connected to a recess part formed in the rigid distal end portion 3c. This recess part is a instrument raising chamber 14, and the instrument raising chamber 14 is open at a portion of the flat portion 11 as an instrument raising opening 14a. The instrument raising chamber 14 is a space for allowing the treatment instrument introduced in the axial direction of the insertion section 3 by the biopsy channel 13 to be oriented toward a direction to be led out of the instrument raising opening 14a, and an instrument raiser member 15 is attached in this instrument raising chamber 14.

The instrument raiser member 15 has a construction which is capable of controlling an angle of the treatment instrument introduced through the biopsy channel 13 into the instrument raising chamber 14 when the treatment instrument is led out of the instrument raising opening 14a. The instrument raising chamber 14 is a longitudinal space in the axial direction of the insertion section 2, and can control the direction in which the treatment instrument protrudes through being elongated toward the axial direction of the instrument raising opening 14a. The instrument raiser member 15 has a guide surface 15a along which the treatment instrument can slide, and is operated to raise up or sink down by remote control from the manipulation section 2 side. In Fig. 3, the position indicated by a solid line is a minimum angular position where the instrument raiser member 15 is sunken down the most, and the treatment instrument is led out diagonally forwards at this time. The position indicated by a dot-chain line is a maximum angular position where the instrument raiser member 15 is raised up to maximum degree, and the treatment instrument is led out in an inclined rearward direction. The range of movement between the minimum angular position and the maximum angular position is an operation range of the instrument raiser member 15 as a raising operation mode. In this way, by inclination and displacement between the minimum angular position and the maximum angular position by the raising operation of the instrument raiser member 15, the lead-out direction of the treatment instrument which is guided along the guide surface 15a of the instrument raiser member 15 is controlled.

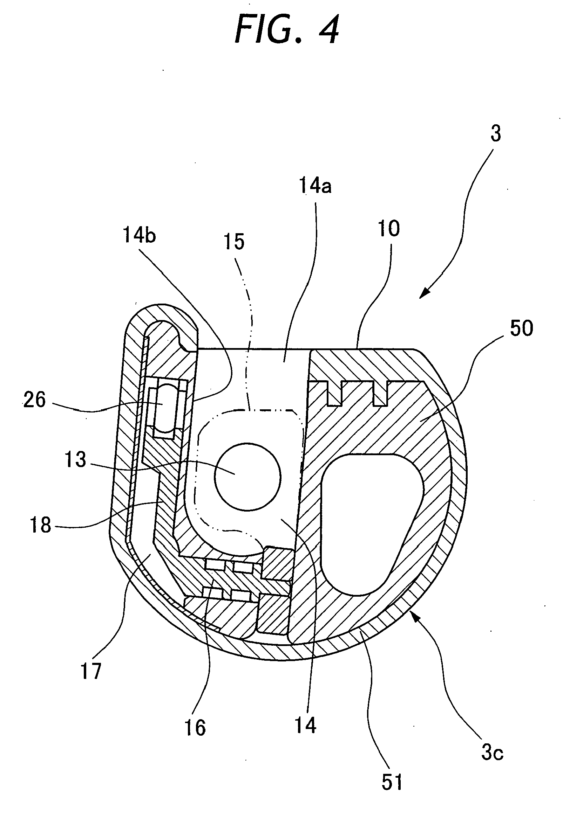

The instrument raiser member 15 is, as shown in Fig. 4, rotatably supported by side wall portion 14b of the instrument raising chamber 14 of the rigid distal end portion 3 by means of a rotational shaft 16. The rotational shaft 16 extends into a lever mounting space 17, and is connected to a driven lever 18 in the lever mounting space 17. The instrument raiser member 15 is rotationally moved around the rotational shaft 16 by means of rotational movement of the driven lever in the forward and rearward direction.

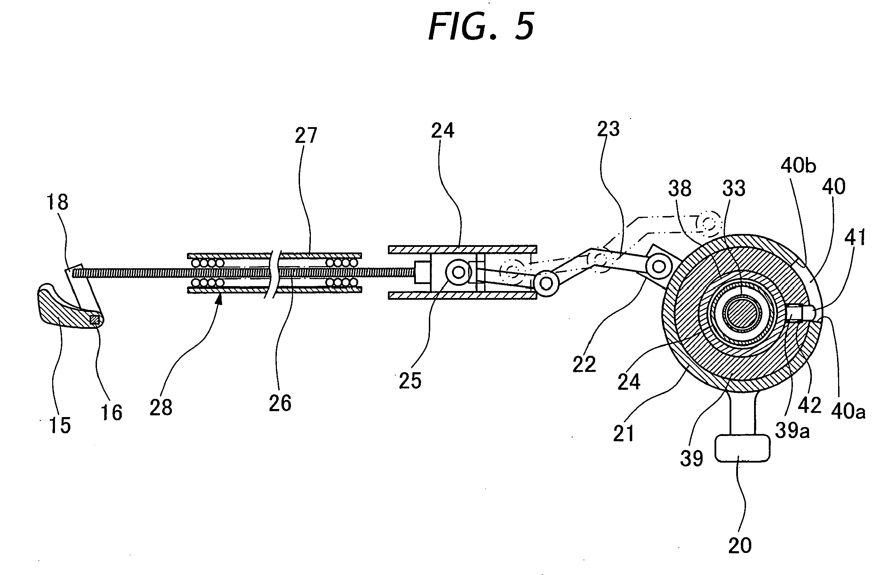

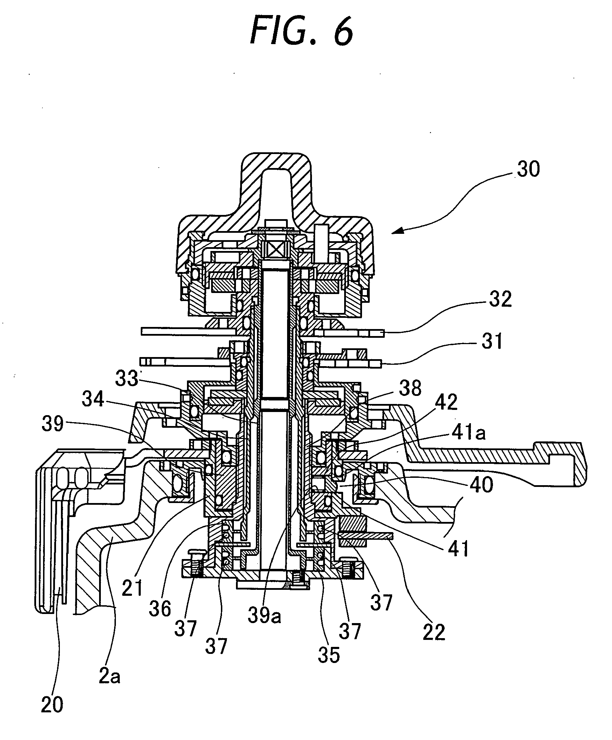

A construction of the raising member operation means, for raising up or sinking down of the instrument raiser member 15 by remote control, is shown in Figs. 5 and 6. A raiser operation lever 20 is disposed on the manipulation section 2, and this raiser operation lever 20 is, as shown in Fig. 5, connected to a rotating drum 21 provided coaxially with hollow rotational shafts 33, 34 of a bending operation means 30, which will be described later. A connecting plate 22 is provided to connect with the rotating drum 21 at one end and is integrated with the raiser operation lever 20. The other end of the connecting plate 22 is pivotally connected to one end of a crank member 23, and the other end of the crank member 23 is pivotally supported by a slider 25 which is slideably disposed on a slide guide 24. An operating wire 26 is connected to the slider 25, and the operating wire 26 is inserted through a flexible sleeve 27. The operating wire 26 and the flexible sleeve 27 constitute a control cable 28. The flexible sleeve 27 can be constituted by an intimately wound coil shaft, and the coil shaft is preferably wrapped with a tube made of heat-shrinkable resin.

As shown in Fig. 6, the bending operation means 30 is disposed on the manipulation section 2 for a bending operation of the movable flexing portion 3b of the insertion section 3 via remote control. The bending operation means 30 has operating knobs 31, 32; one operating knob 31 performs a vertical bending operation of the movable flexing portion 3b, for example, while the other operating knob 32 performs a horizontal bending operation. Coaxially disposed hollow rotational shafts 33, 34 are respectively connected to the operating knobs 31, 32. The hollow rotational shafts 33, 34 are placed in a casing 2a of the manipulation section 2 and are connected to pulleys 35, 36. A pair of operating wires 37 is wound on the pulley 35, 36, respectively. By manipulating the operating knobs 31, 32, the pulleys 35, 36 are rotated to pull in one operating wire 37 and push out the other operating wire 37, and as a result, the movable flexing portion 3b is caused to bend by the operating wire 37 on the pulled side.

The rotating drum 21 of the raiser operation lever 20 is disposed coaxially with the hollow rotational shafts 31, 32 of the bending operation means 30, and a fixing shaft 38 is interposed between the rotating drum 21 and the hollow rotational shaft 32. A restricting drum 39 is connected to the fixing shaft 38 by a screw engagement at a position between the restricting drum 39 and the rotating drum 21, and forms a mechanism for restricting a rotational angle of the rotating drum 21. The raising angle of the instrument raiser member 15, which is varied by operation of the raiser operation lever 20, is limited from a minimum angular position to a maximum angular position.

A groove 40 is formed in the rotating drum 21, and a stopper member 41 is disposed on the restricting drum 39, the stopper member 41 engaging with the groove 40. Therefore, by operating the raiser operation lever 20 so as to rotate the rotating drum 21, it is operated between positions where it contacts the groove end portions 40a, 40b at both ends of the groove 40. If the rotating drum 21 is rotated to the position where the stopper member 41 is brought into contact with the groove end portion 40a of the groove 40, the instrument raiser member 15 is moved to the minimum angular position. Conversely, if it is brought into contact with the other groove end portion 40b, the raiser operation is performed so that the instrument raiser member 15 is located at its maximum angular position. This is an operation range by the raising operation mode.

The rigid distal end portion 3c has a distal end portion main body 50 made of a rigid member, and an outer peripheral portion of the distal end portion main body 50 is covered by a cover member 51. In wall faces constituting the instrument raising chamber 13, a perforation area (or window) is formed in a front end wall 14c (see Fig. 3) located in front of the instrument raiser member 15. This perforation area is disposed in the axial direction of the insertion section 3 and includes a through hole 52 formed in the cover member 51 and, in this embodiment, a transparent plate 53 in the through hole 52.

Within the operation range by the raising operation mode of the instrument raiser member 15, even at the minimum angular position, the instrument raiser member 15 is interposed between the biopsy channel 13 and the transparent plate 53. Thus, the instrument raiser member 15 must be retreated from the position between the biopsy channel 13 and transparent plate 53 so that the forward direction of the biopsy channel 13 can be opened. This constitutes the daughter endoscope passing mechanism, and opens a space at the extended area of the biopsy channel 13 up to the transparent plate 53.

The raiser operation lever 20 can reset the limitation on the operation range of the raising operation mode at both ends of the groove 40 of the rotating drum 21, to allow the instrument raiser member 15 to move to a position of ex-boundary operation mode, in which the instrument raiser member 15 is sunk down to an angle smaller than the minimum angular position. The stopper member 41 is not fixedly disposed on the restricting drum 30, but is fitted in a through hole 39a formed in the restricting drum 39 so as to be retracted into and protruded out from the outer periphery of the restricting drum 39. Further, a spring 42 is mounted to urge the fixing shaft 38 to protrude into the groove 40. The stopper member 41 is formed with a shoulder portion 41 a which is brought into contact with an end portion of the groove 40 of the restricting drum 39, so as to prevent the stopper member 41 from being forced out of the groove 40 by the spring 42.

By forcedly rotating the raiser operation lever in the direction in which the instrument raiser member 15 is brought to a further smaller angle at the time of the stopper member 41 being in contact with the groove end portion 40a of the groove 40 in the rotating drum 21, the stopper member 41 can be pressed down against the urging force of the spring 42, so as to cancel the restriction on the motion of the rotating drum 21 by the stopper member 41. For this purpose, a distal end portion of the stopper member 41 is formed with a spherical shape, so that the stopper member 41 can retract to a position lower than the groove 40 in the rotating drum 21. As a result, the instrument raiser member 15 can be displaced at an angle smaller than the minimum angular position.

As mentioned above, by releasing the restriction on the operation range of the rotating drum 21 by the stopper member 41, the mode is brought into the ex-boundary operation mode, and a position of the instrument raiser member 15 at this time is the retreated position. If the instrument raiser member 15 is displaced to this retreated position, the forward position of the biopsy channel 13 is opened. Also, by returning the raising operation lever 20 to the normal range of the raising operation mode, the stopper member 41 is returned to the state engaged with the groove 40 by the action of the spring 42.

As mentioned above, the instrument raiser member 15 can be displaced to the retreated position, and the through hole 52 is formed at an extended position of the biopsy channel 13, thereby ensuring the forward visual field of the insertion direction in the side-view mother endoscope 1 when the mother endoscope 1 is to be inserted into the body cavity.

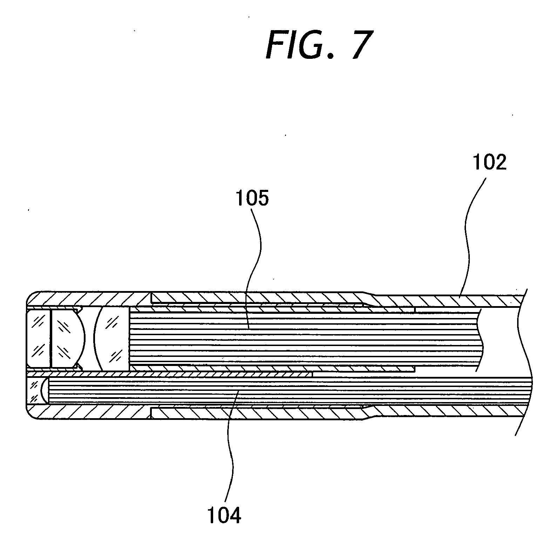

The daughter endoscope 100 is used in order to ensure the forward visual field during the insertion of the mother endoscope 1. The daughter endoscope 100 has a slender insertion section 102 connected to a manipulation section 101 similar to the mother endoscope 1, and a universal cord 103 is connected to the manipulation section 101. Here, the daughter endoscope 100 is, as shown in Fig. 7, an optical endoscope which has a light guide 104 and an image guide 105 in the slender insertion section 102. An ocular portion 106 is disposed at a rear end portion of the manipulation section 101. Although a biopsy channel or a bending operating mechanism is not present, for the sake of reduction of the diameter of the slender insertion section 102 of the daughter endoscope 100, these mechanisms may also be provided. In addition, the daughter endoscope 100 can be formed such that a solid-state image pickup element is attached at an image forming position of the optical system; an image signal from the solid-state image pickup element can then be transmitted and subjected to predetermined signal processing and can be displayed on a monitor screen.

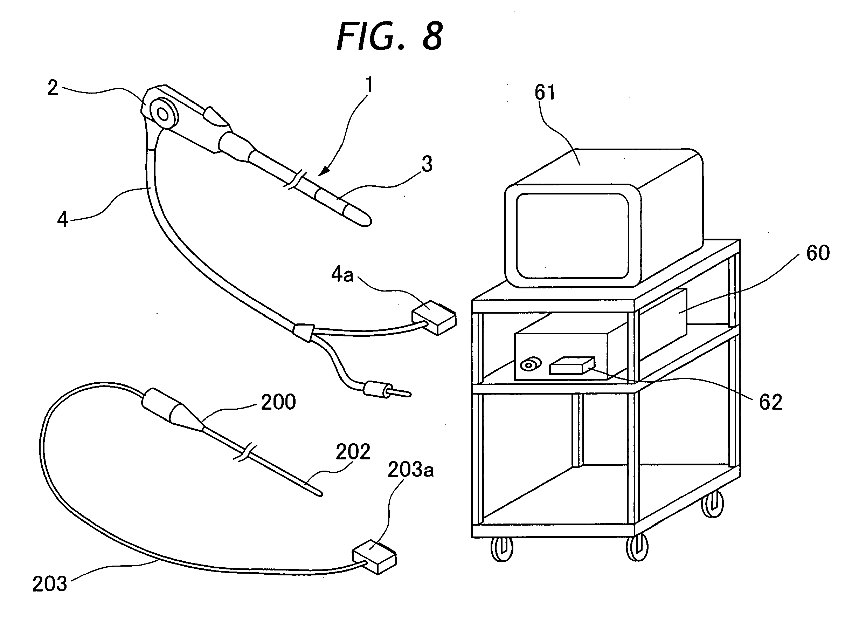

In particular, as shown in Fig. 8, when a daughter endoscope 200 is constituted as an electronic endoscope, a connector 203a can be disposed at an end portion of its universal cord 203. The observation image of the mother endoscope 1 and the observation image of the daughter endoscope 200 can be selectively displayed on a monitor 61 of a processor 60. For that purpose, a construction can be adopted where an electric connection portion 62 of the processor 60 can either be connected to an electric connector 4a disposed at an end portion of the universal cord 4 of the mother endoscope 1 or be connected to the connector 203a disposed at the end portion of the universal cord 203 of the daughter endoscope 200. With such a construction, both a side-view image from the mother endoscope 1 and a straight-ahead image picked up by the daughter endoscope 200 can be displayed on the monitor 61. Therefore, when the connector 4a of the mother endoscope 1 is connected to the connection portion 62, the side-view image from the mother endoscope 1 is displayed on the monitor 61, while when the connector 103a of the daughter endoscope 200 is connected to the connection portion 62, the straight-ahead image from the daughter endoscope 200 is displayed on the monitor 61.

During operation in which the mother endoscope 1 is inserted into the body cavity, the slender insertion section 102 of the daughter endoscope 100 (or the slender insertion section 202 of the daughter endoscope 200) is inserted into the biopsy channel 5 from the manipulation section 2. At the same time, the limitation on the operation range between the stopper member 41 of the restricting drum 39 and the groove 40 in the rotating drum 21 is released, so as to bring the raising operation lever 200 into the ex-boundary operation mode. As a result, the operating wire 26 in the control cable 28 is pushed out of the flexible sleeve 27, the instrument raiser member 15 is displaced to the retreated position, the daughter endoscope passing mechanism is brought into the operating state, and the forward position of the biopsy channel 13 is opened.

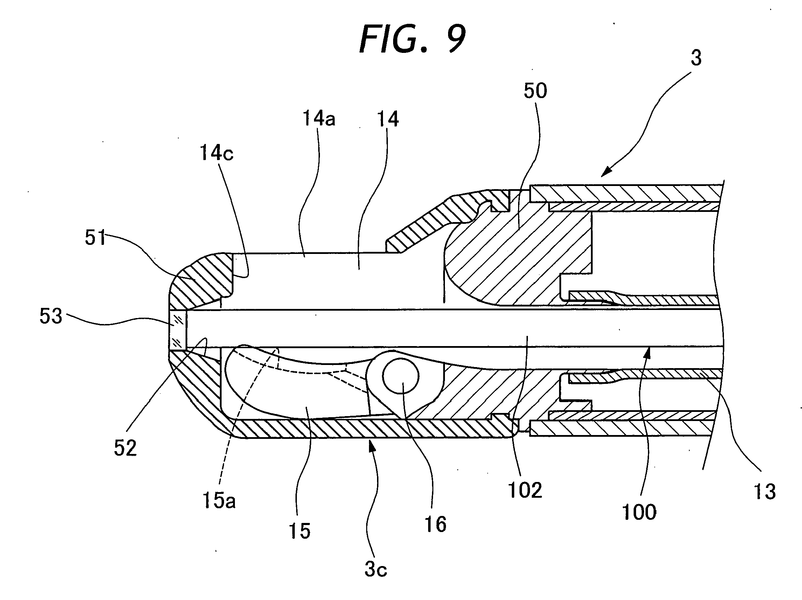

The slender insertion section 102 of the daughter endoscope 100 is introduced from the biopsy channel 13 into the instrument raising chamber 14. Since the through hole 52 is formed in the front end wall 14c of the instrument raising chamber 14, the slender insertion section 102 is inserted into the through hole 52, as shown in Fig. 9. Even if the instrument raiser member 15 does not fully open the through hole 52, in the ex-boundary operation mode, the member is pressed by the end of the slender insertion section 102 so as to retreat from the portion of the through hole 52. The through hole 52 has a taper which expands toward the proximal side, thus ensuring smooth insertion of the slender insertion section 102 into the through hole 52. The transparent plate 53 is disposed in the cover member 51 in the rigid distal end portion 3c, and the transparent plate 53 acts as a restriction portion to prevent the distal end of the slender insertion section 102 protruding from the outer surface of the rigid distal end portion 3c. Thus, the slender insertion section 102 is stopped at the position where it is brought into contact with the transparent plate 53, and does not protrude from the rigid distal end portion 3c. Therefore, the forward visual field can be ensured by the endoscope 100 during insertion of the mother endoscope 1 into a body cavity, thus ensuring quick operation in a safe and smooth manner.

After the flat face portion 10 on which the endoscopic observation means of the rigid distal end portion 3c of the insertion section 3 of the mother endoscope 1 has been introduced into the region where an examination or treatment should be conducted, such as a position of a papilla in a duodenum, the daughter endoscope 100 is separated and withdrawn from the mother endoscope 1. As a result, the biopsy channel 13 can be used for its original function, as a path for introducing a treatment instrument. At this time, since the instrument raiser member 15 is located at the retreated position, the raising operation lever 20 should be operated from the position of the ex-boundary operation mode to the raising operation mode so as to return it within the operation range. The stopper member 41 then returns to engage with the groove 40 of the rotating drum 21 by the force of the spring 42 on the stopper member 41, thereby restricting the operation range of the raising operation lever 20.

Therefore, the mother endoscope 1 can be operated as a usual side viewing endoscope, and an appropriate treatment instrument such as a cannulation tube or a stent can be inserted through the biopsy channel 13. The through hole 52 is formed in the front end wall 14c of the instrument raising chamber 14, and the instrument raiser member 15 is arranged at a position closer to the proximal side from the through hole 52. The treatment instrument is guided by the guide surface 15a of the instrument raiser member 15, and the direction can be changed so that the instrument can be reliably led out of the instrument raising opening 14a. Also, by operating the raising operation lever 20, the treatment instrument led out of the instrument raising opening 14a can be directed to a desired location. Moreover, by inserting the daughter endoscope 100 into the biopsy channel 13, it can be guided by the instrument raiser member 15 in the instrument raising chamber 14 and inserted into the papilla so as to conduct an examination of a biliary duct or the like.

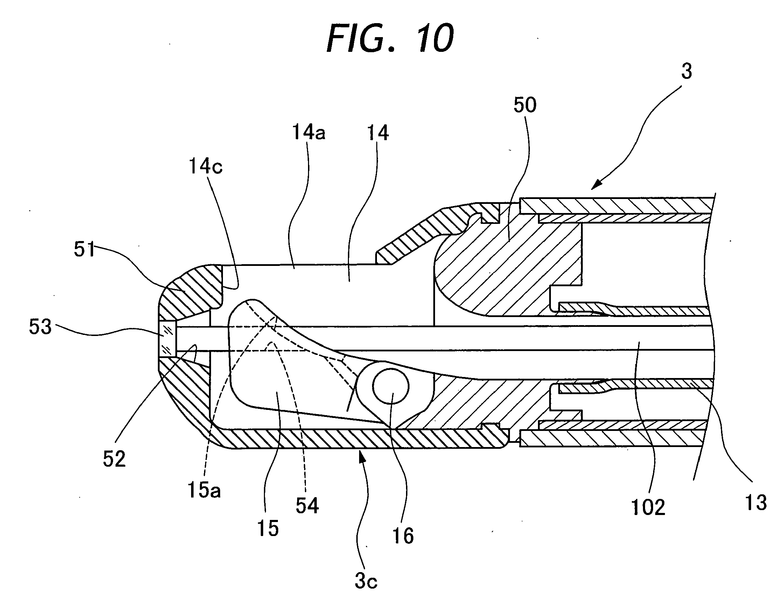

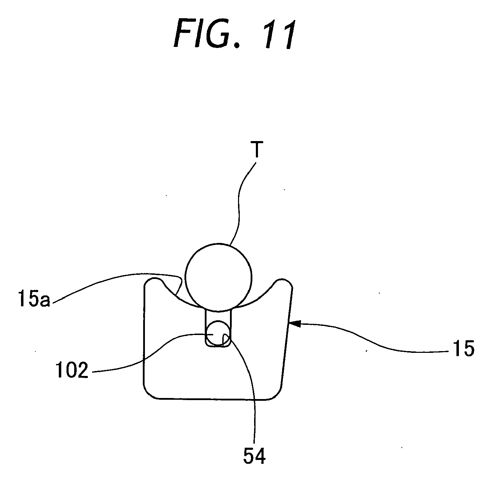

Figs. 10 and 11 show a second embodiment of the present invention. In this second embodiment, the daughter endoscope passing mechanism disposed in the instrument raiser member is constituted by a guide groove 54 which forms a daughter endoscope passage formed in the guide surface 15a of the instrument raiser member 15. This guide groove 54 is an open space from the biopsy channel 13 to the window when the instrument raiser member 15 is held at the minimum angular position of the operation range in the raising operation mode shown in Fig. 10.

The guide groove 54 has a groove width through which the slender insertion section 102 of the daughter endoscope 100 can pass, but which is smaller than an outer diameter dimension of a general treatment instrument. In a side viewing endoscope, which is the mother endoscope 1, various treatment instruments such as a cannulation tube, a stent, forceps, a high-frequency treatment instrument or the like are inserted, and they all have diameters within a certain range. As indicated by a symbol T in Fig. 11, when the treatment instrument having such a diameter is brought into contact with the instrument raiser member 15, the instrument is guided along the guide surface 15a of the instrument raiser member 15. When the slender insertion section 102 of the daughter endoscope 100 is brought into contact with the instrument raiser member 15, it enters the guide groove 54 and advances forward along the guide groove 54. When the slender insertion section 102 is extended to a position opposing the front end wall 14c in the instrument raising chamber 13, it comes into contact with the transparent plate 53, by which the front visual field is ensured by the daughter endoscope 100.

When the mother endoscope 1 is to be inserted into a body cavity, in order to ensure the forward visual field of the insertion section 3 in the mother endoscope 1, the slender insertion potion 102 of the daughter endoscope 100 is inserted into the treatment instrument introduction portion 5 disposed in the manipulation section 2 in advance. Also, the raising operation lever 20 is not operated and is kept at the position where the instrument raiser member 15 is held at the minimum angular position. The distal end of the slender insertion section 102 of the daughter endoscope 100 passes through the guide groove 54 of the instrument raiser member 15 and is introduced into the through hole 52 disposed in the front end wall 14c of the instrument raising chamber 14 at a position such that the distal end of the slender insertion section 102 is brought into contact with the transparent plate 53. As a result, the front visual field of the mother endoscope 1 of the side viewing endoscope is ensured by the daughter endoscope 100, and the insertion operation of the insertion section 3 can be performed safely, smoothly, and quickly.

In short, the front visual field by the daughter endoscope 100 is required for insertion of the mother endoscope 1 into the body cavity at a predetermined position such as inside the duodenum. After the mother endoscope 1 has been inserted to a position where examination or treatment is to be conducted, the daughter endoscope 100 is removed from the biopsy channel 13 so that a required treatment instrument can be inserted in place of the daughter endoscope 100. By providing a shutter to open or close the guide groove 54 as the daughter endoscope passage, the daughter endoscope 100 can be assembled so that it pushes open and passes through the shutter with the distal end portion of its slender insertion section 102 in a state before the mother endoscope 1 is inserted into the body cavity. A construction can be adopted where after the insertion section 3 of the mother endoscope 1 has been inserted to a predetermined position and the daughter endoscope 100 is removed from the biopsy channel 13, the shutter is automatically opened so that the treatment instrument can be inserted smoothly without being blocked.

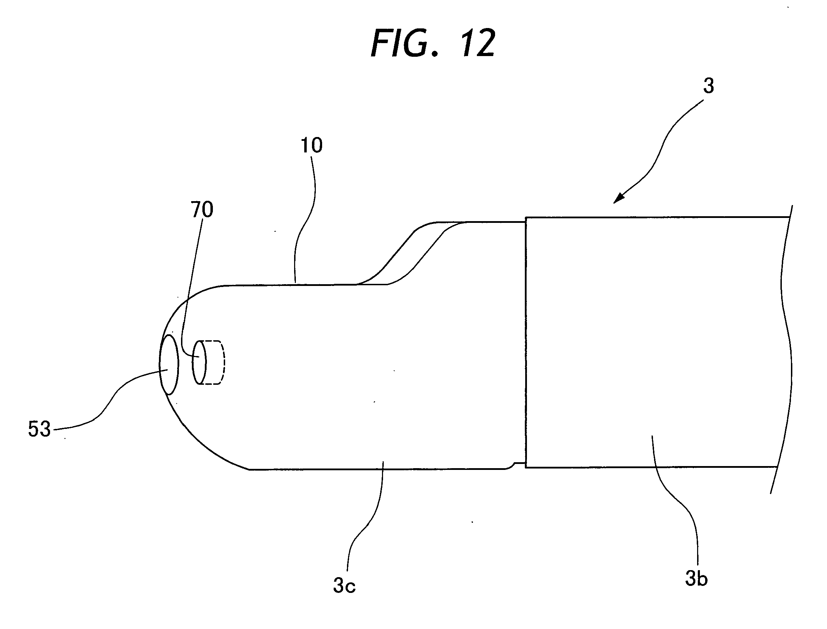

The slender insertion section in the daughter endoscope preferably has as small a diameter as possible. For example, as shown in Fig. 12, it may be so configured that a light-emitting diode or the like, which acts as a light source 70 for emitting illumination light forwards, is mounted in the distal end portion of the rigid distal end portion 3c of the insertion section 3 in the mother endoscope 1. The light source 70 can be made to emit light in the body cavity, which eliminates the need to provide a means for transmitting the illumination light, such as an optical fibre for the daughter endoscope 100; as a result, the diameter of the slender insertion section 102 of the daughter endoscope 100 can be further reduced, or the volume of the image guides 105 can be increased.

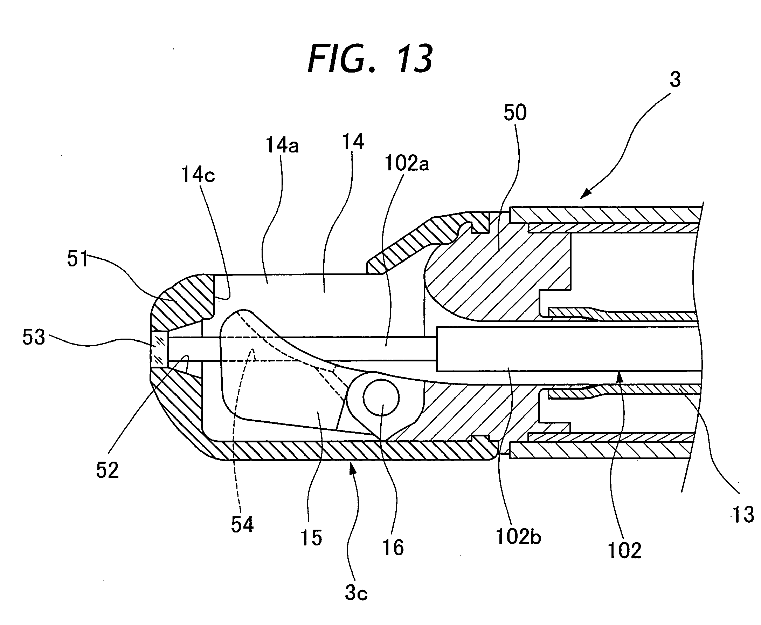

Here, if the diameter of the slender insertion section 102 of the daughter endoscope 100 is reduced, a diameter difference can be provided with respect to the treatment instrument inserted into the biopsy channel 13. However, if the slender insertion section 102 is extremely reduced in diameter, the guidance of the insertion section becomes inferior in the biopsy channel 13 toward the instrument raising chamber 14. As a result, the insertion section may not be introduced into the guide groove 54 formed in the instrument raiser member 15, but might instead be guided along the guide surface 15a toward the direction of the instrument raising opening 14a. Thus, as shown in Fig. 13, a reduced diameter portion 102a of the slender insertion section 102 of the daughter endoscope 100 is limited to a predetermined length on the distal end side, and a portion on the proximal side can be an expanded diameter portion 102b. In this case, the expanded diameter portion 102b has a dimension slightly smaller than the inner diameter of the biopsy channel 13, with the condition that the slender insertion section 102 can smoothly move back and forth in the biopsy channel 13. The reduced diameter portion 102a has a length substantially equal to the distance between a position where the expanded diameter portion 102b is not brought into contact with the instrument raiser member 15 or from the distal end of the biopsy channel 13, for example, to a position where the transparent plate 53 is provided if the distal end of the slender insertion section 102 is located at a position opposing the perforation window 53. The expanded diameter portion 102b may be configured such that the diameter of the slender insertion section 102 is increased, or a cylindrical member may be attached to a portion corresponding to the expanded diameter portion 102b when the diameter of the slender insertion section 102 has a uniform outer diameter.

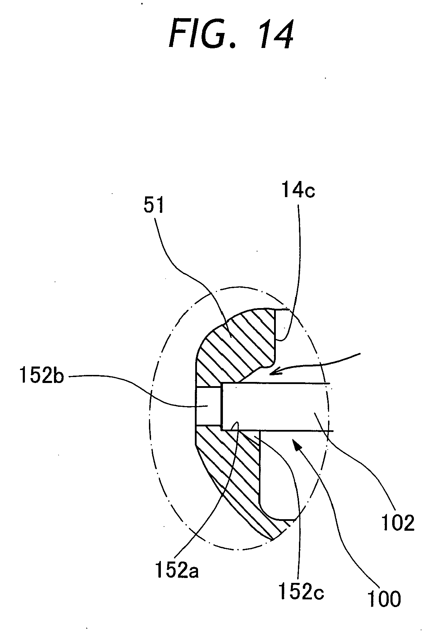

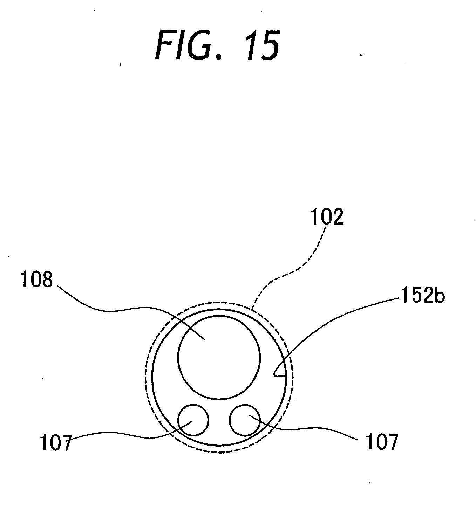

In the first embodiment, as the regulating portion for regulating the slender insertion section 102 of the slender endoscope 100 so that it does not protrude from the through hole 52 formed in the rigid distal end portion 3c in the insertion section 3 of the mother endoscope 1, the transparent plate 53 is provided; however, a construction as shown in Fig. 14 can be provided instead. In the construction in Fig. 14, in the rigid distal end portion 3c, in the front end wall 14c located in front of the instrument raising chamber 14, the through hole 152 formed in the cover member 51 has a stepped structure. That is, at the side of the front end wall 14c is a large diameter portion 152a of the through hole 152, while the diameter of the through hole 152 at the side of the cover member 51 is reduced so as to form a smaller diameter portion 152b. The smaller diameter portion 152b has an inner diameter less than the outer diameter of the slender insertion section 102 of the daughter endoscope 100. A tapered portion 152c is preferably formed on the proximal side of the large diameter portion 152a as necessary. However, in the slender insertion section 102 of the daughter endoscope 100, it is necessary that an illumination lens 107 and an objective lens 108 which may be present at the distal end should not be covered by the stepped face. Thus, as shown in Fig. 15, a diameter difference between the inner diameter of the slender portion 152b and the outer diameter of the slender insertion section 102 should be extremely small. As a result, any reduction in the amount of illumination light or limitation on the observation visual field will not occur or can be minimized. If the outer diameter of the slender insertion section 102 is smaller than the inner diameter of the through hole 152, the distal end portion of the slender insertion section 102 may be wrapped with medical tape or the like so that the slender insertion section 102 does not pass through the through hole 152.

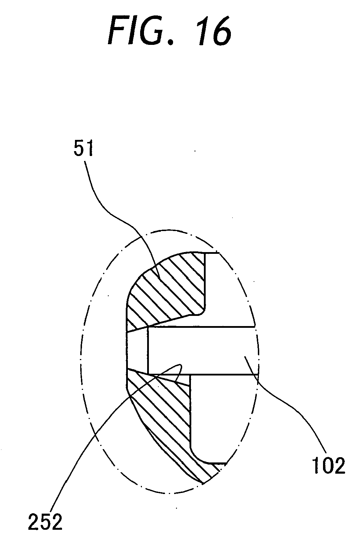

Also, the configuration of the through hole may be a through hole 252 as shown in Fig. 16, in the form of a tapered hole whose diameter continuously reduces toward the distal side thereof. In this case, the smallest diameter of the through hole 252 should be slightly smaller than the outer diameter of the slender insertion section 102.

## Claims

1. A side viewing endoscope system comprising:
a mother endoscope (1) of a side viewing type, in which an observation means having an illumination portion (11) and an observation portion (12) is disposed on a side surface (10) of a rigid distal end portion (3c) of an insertion section (3), a instrument raising chamber (14) including an instrument raiser member (15) for raising up or sinking down of a treatment instrument is formed in said rigid distal end portion (3c), a biopsy channel (13) provided in an axial direction of said insertion section (3) being in communication with said instrument raising chamber (14); and
a daughter endoscope (100) having a slender insertion section (102) which can be inserted into said biopsy channel (13) of said mother endoscope (1) and having a viewing field in a forward direction of a distal end of said slender insertion section (102);
wherein a window (52, 53) is formed at said rigid distal end portion (3c) of said mother endoscope (1) having a viewing field toward the forward direction of said biopsy channel (13); and
said distal end portion of said daughter endoscope (100) may be introduced through said biopsy channel (13) into a position facing said window (52, 53) but not protruding from the outer surface of said rigid distal end (3c) of said mother endoscope (1).

2. A side viewing endoscope system according to claim 1, wherein said instrument raiser member (15) disposed in said instrument raising chamber (14) is provided with a daughter endoscope passing mechanism to allow said slender insertion section (102) of said daughter endoscope (100) to pass therethrough.

3. A side viewing endoscope system according to claim 2, wherein said instrument raiser member (15) is mounted on a rotatable shaft (16) in said rigid distal end portion (3c), a raiser operating means (20) is provided on a manipulating section (2) of said mother endoscope (1) for raising up or sinking down said instrument raiser member (15), and said daughter endoscope passing mechanism is adapted to shift said instrument raiser member (15) to a retreated position.

4. A side viewing endoscope system according to claim 3, wherein said raiser operating means (20) is adapted to change over between a raising operation mode, to move said instrument raiser member (15) between a minimum angular position and a maximum angular position, and an ex-boundary operation mode, in which said instrument raiser member (15) is displaced to said retreated position, and
wherein said instrument raiser member (15) is normally operated in said raising operation mode.

5. A side viewing endoscope system according to claim 2, wherein a daughter endoscope passage (54) is formed on a guide surface (15a) of said instrument raiser member (15) for guiding said treatment instrument.

6. A side viewing endoscope system according to claim 5, wherein said daughter endoscope passage has a guide groove (54), the width of the guide groove (54) being smaller than an outer diameter of said treatment instrument to be inserted into said biopsy channel of said mother endoscope (1) and being larger than a diameter of said slender insertion section (102) of said daughter endoscope (100).

7. A side viewing endoscope system according to claims 1 to 6, wherein said window is formed as a through hole (52) in a front end wall of said rigid distal end portion (3c) of said mother endoscope (1), said slender insertion section (102) can be inserted into said through hole (52), and said through hole (52) has a restricting means to prevent said slender insertion section (102) from protruding out of an outer end surface of said rigid distal end portion (3c).

8. A side viewing endoscope system according to claim 7, wherein said restricting means is constituted by a transparent plate (53) in said through hole (52).

9. A side viewing endoscope system according to claim 7, wherein said restricting means is formed of a portion with a stepped wall (152b) having a diameter smaller than the outer diameter of said slender insertion section (102).

10. A side viewing endoscope system according to claim 7, wherein said through hole (252) has a taper portion whose diameter is continuously reduced toward the front side, and said restricting means is constituted by the reduced smallest diameter portion of said taper hole which is smaller than the outer diameter of said slender insertion section (102).

11. A side viewing endoscope system according to any one of claims 1 to 10,
wherein said mother endoscope (1) and said daughter endoscope (100) are electronic endoscopes, and a front view observation image can be displayed on a monitor screen (61) when said daughter endoscope (100) is inserted into said biopsy channel (13), and a side view observation image can be displayed on said monitor screen (61) when said daughter endoscope (100) is removed.
